# FASCICULE DE BREVET EUROPEEN

(11) **EP 1 309 364 B1**
(45) Date de publication et mention de la délivrance du brevet: **11.07.2007**
(21) Numéro de dépôt: 01965322.9
(22) Date de dépôt: 10.08.2001
(51) Int. Cl.: A61M 1/02, B65G 49/00

(54) **PROCEDE ET INSTALLATION DE MANIPULATION AUTOMATIQUE DE SYSTEMES A POCHES**
VERFAHREN UND EINRICHTUNG ZUR AUTOMATISCHEN HANDHABUNG VON BEUTELSYSTEMEN
METHOD AND INSTALLATION FOR AUTOMATIC HANDLING OF BAG SYSTEMS

(30) Priorité: 10.08.2000 FR 0010534
(43) Date de publication de la demande: 14.05.2003
(73) Titulaire: MACO PHARMA, 59420 Mouvaux (FR)
(72) Inventeur: GOUDALIEZ, Francis, F-59155 Faches-Thumesnil (FR); VERPOORT, Thierry, F-59420 Mouvaux (FR)
(74) Mandataire: Geismar, Thierry
(86) Numéro de dépôt international: PCT/FR2001/002602
(87) Numéro de publication internationale: WO 2002/011788

(56) Documents cités:
- EP-A- 1 048 305
- WO-A-94/01193
- FR-A- 2 581 044
- US-A- 4 386 504

## Description

L'invention est relative à un procédé de manipulation automatique d'au moins un système à poches dans une installation ainsi qu'à ladite installation.

Elle s'applique typiquement au cas où un fluide, notamment biologique, par exemple du sang ou un composant du sang, après avoir été reçu dans une poche primaire d'un système à poches doit être filtré.

A cet effet, le système à poches comprend en plus de la poche primaire et reliées à elle en série par l'intermédiaire de tubulures, au moins une unité de filtration et au moins une poche secondaire destinée à recevoir le filtrat.

Il est connu de réaliser la filtration en manipulant, notamment en homogénéisant le contenu de la poche primaire et en la retournant manuellement, le système à poches de sorte que le fluide s'écoule par gravité depuis la poche primaire vers la poche secondaire par l'intermédiaire de l'unité de filtration.

Mais lorsqu'un grand nombre de tels systèmes à poches doit être manipulé cette méthode devient vite fastidieuse.

Par ailleurs, dans le domaine de la filtration sanguine, le temps entre le recueil du sang et sa filtration doit être contrôlé de façon précise afin que le filtrat soit de bonne qualité.

La méthode manuelle précédemment exposée ne permet pas ce contrôle précis, notamment pour un grand nombre de système à poches.

De plus l'intervention humaine est source d'erreurs et ne permet pas une tracabilité satisfaisante des systèmes à poches et de leur contenu, notamment en terme de délai entre le prélèvement et la filtration ainsi qu'en terme de durée de la filtration.

Le document WO94/01193 décrit un système automatisé pour le traitement d'un fluide biologique. Le système comprend un générateur différentiel de pression, un ensemble à poches comprenant des filtres à déleucocyter, et un dispositif de contrôle pour contrôler le flux de fluide dans l'ensemble à poches. Dans ce système, la filtration du fluide est réalisée sous pression.

EP-A-1 048 305 décrit un système pour la filtration du sang où une poche secondaire est exposée à un pressurage après que la poche a été remplie de sang.

Le document est pertinent seulement selon l'Article 54(3) CBE.

L'invention vise donc à remédier à ces inconvénients en proposant un procédé de manipulation de système à poches dans une installation qui soit compacte eu égard au nombre de systèmes manipulés, dans lequel l'intervention humaine est limitée au chargement et au déchargement des systèmes à poches respectivement avant et après la filtration. et dans lequel la manipulation des systèmes à poches est réalisée de façon automatique en permettant le contrôle du temps entre le recueil et la filtration du fluide.

A cet effet, selon un premier aspect l'invention propose un procédé de manipulation automatique d'au moins un système à poches dans une installation, ledit système à poches comprenant au moins une poche primaire contenant un fluide, notamment biologique, par exemple du sang ou un composant du sang, au moins une unité de filtration et au moins une poche secondaire destinée à recevoir le filtrat, ledit procédé comprenant les étapes de :
- chargement d'au moins un système à poches dans un dispositif de manipulation, ledit système à poches étant dans une position telle que la filtration du fluide n'a pas lieu ;
- actionnement automatique du dispositif de manipulation de sorte que le système à poches soit manipulé vers une position dans laquelle le fluide s'écoule par gravité depuis la poche primaire vers la poche secondaire par l'intermédiaire de l'unité de filtration, de sorte à permettre la réalisation de la filtration du fluide à un instant prédéterminé ;
- déchargement du système à poches présent dans le dispositif de manipulation lorsque tout le filtrat a été reçu dans la poche secondaire.

Selon une réalisation, le procédé comprend, préalablement à l'actionnement du dispositif de manipulation, une étape de stockage du système à poches associé audit dispositif pendant un temps prédéterminé. Par exemple, l'étape de stockage est réalisée par transfert du système à poches associé audit dispositif de manipulation vers une première zone de stockage.

Selon une réalisation, l'étape d'actionnement du dispositif de manipulation est précédée par une étape d'agitation du contenu du système à poches, notamment de celui de la poche primaire. Par exemple, l'étape d'agitation est réalisée par actionnement partiel du dispositif de manipulation.

Selon une réalisation, le procédé comprend, postérieurement à l'actionnement du dispositif de manipulation, une étape de stockage du système à poches associé audit dispositif pendant le temps nécessaire à la filtration de sensiblement la totalité du fluide. Par exemple, l'étape de stockage est réalisée par transfert du système à poches associé audit dispositif de manipulation vers une deuxième zone de stockage.

En variante, la fin de l'étape de stockage est commandée par des moyens de détermination de l'achèvement de la filtration.

Selon une réalisation, le procédé comprend en outre une ou des étapes d'identification du système à poches et/ou du dispositif de manipulation présent dans l'installation. Par exemple, l'étape d'identification comprend la lecture et/ou l'enregistrement d'informations concernant le dispositif de manipulation, le système à poches, le contenu de la poche primaire, le personnel ayant utilisé le système à poches.

En variante, les informations sont contenues dans un dispositif électronique associé au dispositif de manipulation et/ou au système à poches, la succession des différentes étapes pouvant être commandée en fonction des informations lues lors de l'étape d'identification.

En variante, une étape d'identification est réalisée lors du chargement du système à poches dans l'installation de sorte notamment à lire des informations concernant le contenu de la poche primaire, l'heure d'actionnement du dispositif de manipulation pouvant être commandé en fonction des informations lues lors de l'étape d'identification, notamment en fonction de l'heure de prélèvement. En outre, l'actionnement du dispositif de manipulation peut également être commandé en fonction de la température du fluide à filtrer.

En variante, une étape d'identification est réalisée lors du déchargement du système à poches de sorte notamment à enregistrer des informations concernant la réalisation de la filtration.

Selon une réalisation, une pluralité de systèmes à poches est manipulée dans un même dispositif de manipulation, par exemple entre 10 et 30 systèmes à poches et une pluralité de système à poches est manipulée simultanément dans une même installation, par exemple entre 500 et 5000 systèmes à poches.

Selon un deuxième aspect, l'invention propose une installation pour la mise en oeuvre du procédé décrit ci-dessus, qui comprend:
- un dispositif de manipulation apte à recevoir au moins un système à poches
- des moyens d'actionnement aptes à actionner ledit dispositif de manipulation depuis une position dans laquelle la filtration du fluide présent dans le système à poches associé n'a pas lieu vers une position dans laquelle la filtration du fluide a lieu;
- une zone de chargement du système à poches dans le dispositif de manipulation ;
- une zone de déchargement du système à poches associé au dispositif de manipulation ;
- une zone d'actionnement du dispositif de manipulation dans laquelle les moyens d'actionnement sont prévus ;
ladite installation comprenant au moins une voie de circulation principale motorisée sur laquelle le dispositif de manipulation est déplacé depuis la zone de chargement vers la zone de déchargement via la zone d'actionnement et au moins une unité de commande des déplacements du dispositif de manipulation.

Selon une variante, les zones de chargement et de déchargement sont les mêmes.

Selon une réalisation, l'installation comprend en outre, une première et une deuxième zones de stockage dans laquelle le système à poches associé au dispositif de manipulation est stocké respectivement avant et pendant la filtration. Les zones de stockage peuvent alors être formées par au moins un tronçon de voie de circulation secondaire motorisée.

Selon une réalisation, l'installation comprend en outre une zone d'agitation du contenu du système à poches, ladite zone d'agitation étant pourvue de moyens d'agitation. Par exemple, la zone d'agitation est identique à celle d'actionnement du dispositif de manipulation, les moyens d'actionnement formant également moyens d'agitation.

Selon des variantes, l'installation peut comprendre en outre:
- des moyens de détermination de l'achèvement de la filtration, lesdits moyens de détermination étant reliés à l'unité de commande;
- des moyens d'identification du système à poches et/ou du dispositif de manipulation apte à lire et/ou à écrire des informations dans un dispositif électronique associé audit système à poches et/ou audit dispositif de manipulation, lesdits moyens d'identification étant reliés à l'unité de commande;
- des moyens de détermination de température du fluide à filtrer, lesdits moyens de détermination étant reliés à l'unité de commande.

D'autres objets et avantages de l'invention apparaîtront au cours de la description qui suit en référence aux dessins annexés.

La figure 1 représente, en vue de dessus et de façon schématique, la structure fixe d'une installation de manipulation automatique selon l'invention comprenant une voie de circulation principale motorisée qui est réalisée en boucle fermée et neuf tronçons de voie secondaire motorisée qui sont disposés sensiblement perpendiculairement à la grande longueur de ladite boucle.

La figure 2 représente, en vue de coté et de façon schématique, un mode de réalisation d'un dispositif de manipulation de dix systèmes à poches comprenant chacun une poche primaire, une unité de filtration et une poche secondaire, ledit dispositif étant suspendu à la voie de circulation principale.

La figure 3 est une vue de profil du dispositif de manipulation représenté sur la figure 2.

La figure 4 représente, en vue de coté et de façon schématique, deux dispositifs de manipulation analogues à ceux de la figure 2 qui se déplacent conjointement et l'un derrière l'autre sur la voie de circulation principale.

La figure 5 représente, en vue de coté et de façon schématique, cinq dispositifs de manipulation analogues à ceux de la figure 2 qui se trouvent sur un tronçon de voie secondaire formant zone de stockage.

La figure 6 représente, en vue de dessus et de façon schématique, un mode de réalisation des moyens d'agitation du contenu des systèmes à poches associés à un dispositif de manipulation selon la figure 2.

Les figures 7a à 7c représentent, en vue de coté et de façon schématique, un mode de réalisation des moyens d'actionnement aptes à actionner un dispositif de manipulation selon la figure 2 depuis une position dans laquelle la filtration des fluides présents dans les systèmes à poches associés n'a pas lieu vers une position dans laquelle la filtration des fluides a lieu, ainsi que trois étapes de cet actionnement.

Une installation de manipulation automatique selon l'invention comprend une structure fixe 1 et des dispositifs de manipulation 2 dans lesquels les systèmes à poches 3 sont reçus.

Les dispositifs de manipulation 2 sont associés à la structure fixe 1 de sorte à pouvoir être d'une part déplacés le long de ladite structure 1 et d'autre part actionnés pour permettre la filtration du fluide.

Dans le mode de réalisation représenté sur les figures, le convoyage des dispositifs de manipulation 2 le long de la structure fixe 1 est réalisée de façon aérienne, c'est à dire que les dispositifs de manipulation 2 sont suspendus à la structure fixe 1 sans toucher le sol 4 sur lequel repose ladite structure fixe 1.

Toutefois, un autre mode de convoyage est également envisageable, notamment en disposant les dispositifs de manipulation 2 sur un convoyeur, par exemple du type à bande sans fin, ou encore en prévoyant que les dispositifs de manipulation 2 puissent se déplacer sur le sol, par exemple au moyen de roues.

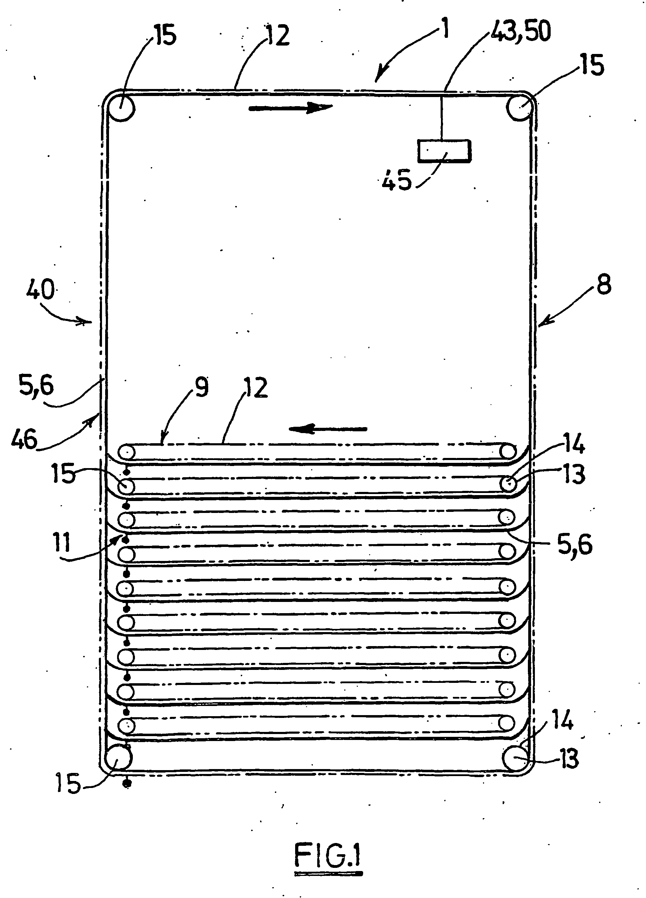

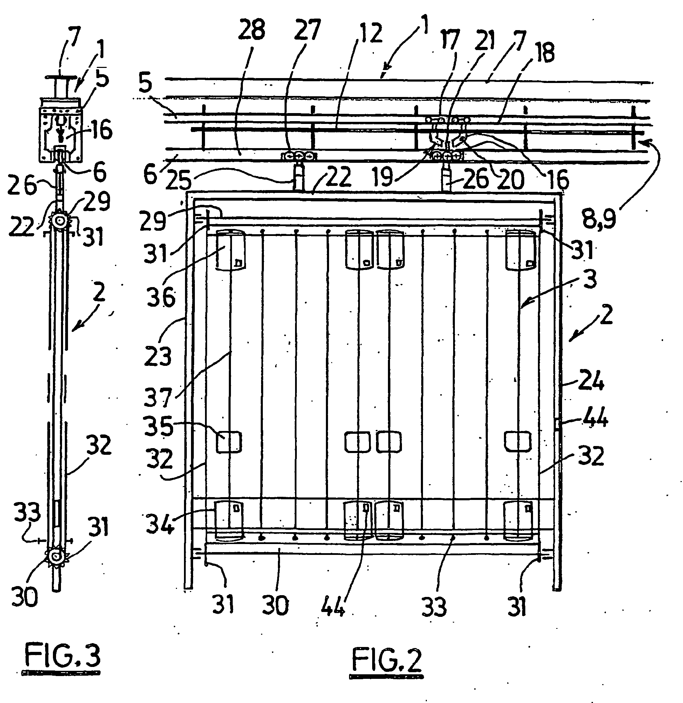

La structure fixe 1 représentée notamment sur les figures 1 et 2, comprend deux types de rail dont le premier 5 est disposé sensiblement au-dessus du second 6. Les deux types de rail 5, 6 sont associés à une distance définie l'un de l'autre et sont portés par une structure porteuse 7. Le premier type de rail 5 a pour fonction de permettre l'entraînement des dispositifs de manipulation 2, il est donc dit « tracteur », alors que le deuxième type 6 a pour fonction de supporter en suspension les dispositifs de manipulation 2, il est donc dit « porteur ».

Les rails des premier et deuxième types 5, 6 sont par exemple formés en acier ou analogue de sorte à pouvoir supporter les efforts engendrés par le poids et le convoyage des dispositifs de manipulation 2. Les rails 5, 6 sont soit de forme rectiligne soit de forme curviligne et sont associés entre eux, par exemple par soudage, pour former des voies de circulation et d'entraînement pour les dispositifs de manipulation 2.

Dans le mode de réalisation représenté sur la figure 1, la structure fixe 1 comprend une voie de circulation principale motorisée 8 qui est réalisée en boucle fermée et neuf tronçons de voie secondaire motorisée 9 qui sont disposés sensiblement perpendiculairement à la grande longueur de ladite boucle.

La structure fixe 1 est maintenue à une hauteur supérieure à celle des dispositifs de manipulation 2 au moyen de poteaux 10 disposés sur le sol 4, de sorte que lesdits dispositifs 2 puissent circuler librement sans toucher le sol 4.

Bien que cette réalisation ne soit pas spécifiquement décrite ici, la structure fixe 1. peut comprendre plus d'une voie principale 8 et un nombre de voies secondaires 9 différent, éventuellement disposées différemment les unes par rapport aux autres.

En effet, l'homme du métier peut ajuster l'architecture de la structure fixe 1 en fonction de ses contraintes spécifiques, notamment le nombre de dispositifs de manipulation 2, la nature des fluides à filtrer, la présence de zones de stockage, l'emplacement où l'installation doit être montée.

Dans la réalisation décrite, les dispositifs de manipulation 2 circulant sur la voie principale 8 peuvent être déviés au moyen d'un système d'aiguillage 11 vers l'une des voies secondaires 9, et vice versa.

A cet effet, un système d'aiguillage 11 est prévu à chaque intersection entre une voie secondaire 9 et la voie principale 8, c'est à dire à chaque extrémité de chacune des voies secondaires 9.

On décrit ci-dessous la motorisation des voies de circulation principale 8 et secondaires 10 au moyen d'un dispositif d'entraînement associé au rail tracteur 5.

Dans le mode de réalisation représenté sur la figure 2, ce dispositif comprend un câble 12 en boucle fermée qui est entraîné en translation, de façon sensiblement parallèle au rail tracteur 5, au moyen d'un moteur 13.

A cet effet, la voie principale 8 comprend une poulie motorisée 14 et trois poulies de renvoi 15 et chacune des voies secondaires 9 une poulie motorisée 14 et une poulie de renvoi 15. La circulation des dispositifs de manipulation 2 le long de la voie principale 8 est alors circulaire tandis que celle le long d'une voie secondaire 9 est linéaire entre deux points de la voie principale 8, respectivement dans le sens indiqué par les flèches de la figure 1.

Des mobiles 16 sont associés, notamment de façon fixe, au câble 12 de sorte à être déplacé par lui. Les mobiles 16 comprennent des roues 17 qui sont disposées dans une gorge 18 du rail tracteur 5 de sorte que le mobile 16 soit guidé dans ses déplacements. D'autre part le mobile 16 comprend des moyens de préhension 19 s'étendant en direction du rail porteur 6 et qui sont aptes à venir engager une pièce complémentaire associée à un dispositif de manipulation 2 de sorte à transférer le mouvement du câble 12 audit dispositif 2.

Comme représenté notamment sur la figure 2, les moyens de préhension 19 sont formés de doigts escamotables 20 qui sont actionnés depuis une position où ils sont engagés sur un téton 21 d'un dispositif de manipulation 2 vers une position où ils sont désengagés, et vice versa.

Les moyens de préhension 19 peuvent être actionnés par un système de came (non représenté) qui en venant en appui sur une pièce du mobile permet de déplacer les doigts escamotables 20 de sorte à les engager/désengager du téton 21.

Dans cet exemple, les systèmes d'aiguillage 11 comprennent les systèmes de came de sorte à pouvoir désengager / engager les tétons 21 des dispositifs de manipulation 2 de la voie de circulation principale 8 lors du changement de voie.

Le nombre et/ou la position des mobiles 16 sur le câble 12 est défini notamment en fonction du nombre de dispositifs de manipulation 2 et de la nature des déplacements souhaités. Par exemple, les mobiles 16 peuvent être disposés à équidistance les uns des autres de sorte que la distance entre deux dispositifs 2 successifs soit définie et fixe. Cette distance peut être différente en fonction des voies considérées, notamment entre la voie principale 8 et les voies secondaires 9.

On décrit ci-dessous, en relation notamment avec les figures 2 et 3, un mode de réalisation d'un dispositif de manipulation 2 suivant l'invention.

Le dispositif de manipulation 2 comprend un cadre formé de trois traverses par exemple en acier ou analogue, respectivement supérieure 22, gauche 23 et droite 24, s'étendant sur trois cotés d'un rectangle.

Deux suspentes 25, 26 sont associées fixement sur la face externe de la traverse supérieure 22 de sorte à permettre l'accrochage du dispositif de manipulation 2 sur le rail porteur 6 de la structure fixe 1.

A cet effet, l'extrémité des suspentes opposée à la traverse supérieure 22 est pourvue de roues 27 qui sont disposées dans une gorge 28 du rail porteur 6 de sorte à guider les déplacements du dispositif de manipulation 2.

Un téton 21 est prévu sur l'une de ces suspentes 26, au voisinage de ces roues 27 et de façon saillante de la gorge 28 du rail porteur 6, de sorte à permettre son engagement avec les moyens de préhension 19 du mobile 16 tel que décrit ci-dessus.

Dans le mode de réalisation, la suspente 26 comprenant le téton 21 est donc active vis à vis du déplacement du dispositif de manipulation 2 alors que l'autre suspente 25 est passive. On peut toutefois prévoir un nombre de suspentes 25, 26 actives et/ou passives différent.

A l'intérieur du cadre, et associés d'une part à la traverse gauche 23 et d'autre part à la traverse droite 24, sont prévus deux arbres 29, 30 mobiles en rotation. Les deux arbres 29, 30 sont sensiblement parallèles et sont espacés d'une distance D.

A chaque extrémité des arbres 29, 30 est prévue une roue dentée 31 apte à recevoir une chaîne 32 de sorte à asservir le mouvement de rotation de chacun des deux arbres 29, 30. Bien que le mode de réalisation décrit comprend deux chaînes 32 et quatre roues dentées 31, seulement une chaîne 32 et deux roues dentées 31 peuvent être prévues ou un autre dispositif d'asservissement du mouvement de rotation des deux arbres 29, 30 peut être imaginé par l'homme du métier. Le dispositif de manipulation comprend des moyens de fixation 33 des systèmes à poches 3. Par exemple, il est classique que les poches d'un système à poches 3 comprennent des oeillets dans lesquels une saillie formant moyen de fixation 33 peut être introduite.

Dans le mode de réalisation représenté, dix moyens de fixations 33 sont prévus en regard les uns des autres, de sorte à pouvoir associer dix systèmes à poches 3 dans chaque dispositif de manipulation 2.

Les systèmes à poches 1 comprennent une poche primaire contenant un fluide, notamment biologique, par exemple du sang ou un composant du sang, au moins une unité de filtration 35 et au moins une poche secondaire 36 destinée à recevoir le filtrat, les poches 34, 36 et l'unité de filtration 35 étant relié entre eux par l'intermédiaire de tubulures 37.

L'installation permet alors de réalisé, en circuit clos, la filtration et le recueil du fluide filtré lorsque le dispositif de manipulation 2 est actionné.

Lors du chargement d'un système à poches 1, la poche primaire 34 est associée au dispositif de filtration par l'intermédiaire de respectivement un moyen de fixation 33 (voir figure 2). Dans cette position, la filtration du fluide par gravité n'a donc pas lieu.

Dans le mode de réalisation représenté, les dix systèmes à poches 3 sont disposés sensiblement parallèlement les uns par rapport aux autres avec leur unité de filtration 35 disposée entre les deux arbres 29,30, l'espace défini entre les deux arbres 29, 30 étant vide. On peut toutefois prévoir qu'une bande sans fin y soit disposée pour soutenir les unités de filtration 35.

Les systèmes à poches 3 peuvent être identiques et/ou différents, en particulier ils peuvent comprendre d'autres poches, d'autres unités de filtration ou toutes autres dispositifs nécessaires au traitement du fluide avant et/ou après sa filtration.

Dans un exemple particulier, la poche primaire 34 contient du sang total et l'unité de filtration 35 est apte à éliminer les leucocytes. Le système à poches 3 peut alors comprendre en outre une première et une deuxième poches satellites reliées en série à la poche secondaire. Dans cette réalisation la filtration est réalisée dans une installation objet de l'invention puis, après déchargement du système à poches 3, la poche secondaire 36 est centrifugée de sorte à recueillir le concentré globulaire et le plasma sanguin respectivement dans la première et dans la deuxième poche satellite.

En fonction de la nature et/ou de la géométrie des systèmes à poches 3 à disposer dans les dispositifs de manipulation 2, la distance D peut être réglable, notamment en prévoyant des moyens de réglage aptes à faire coulisser l'un des arbres 29, 30 par rapport à l'autre.

On décrit ci-dessous l'actionnement d'un tel dispositif de manipulation 2 au moyen d'un premier 38 et d'un deuxième vérins 39 sans tige, par exemple du type pneumatique ou pignon crémaillère (voir figure 7a à 7c).

Les vérins 38, 39 sont disposés dans une zone de l'installation, dite zone d'actionnement 40 du dispositif de manipulation 2, dans laquelle les moyens de préhension 19 sont désengagés de sorte que ledit dispositif 3 soit immobile.

Sur les chaînes 32 sont prévus des moyens formant prises pour la tige 42 des vérins 38, 39, par exemple sous la forme de butées 41a, 41b, saillants du dispositif de manipulation 2. Les butés 41 a, 41b sont par exemple au nombre de deux et sont disposées sur l'une des chaînes 32 à une distance égale à approximativement D.

La figure 7a montre le dispositif de manipulation 2 dans une position où la filtration du fluide n'a pas lieu. Dans cette position, la poche primaire 34 est disposée au-dessous de la poche secondaire 36 de sorte que l'écoulement du fluide entre ces deux poches 34, 36 ne puisse pas se produire par gravité.

Pour actionner le dispositif de manipulation 2, le premier vérin 38 vient s'engager sur la butée supérieure 41a de sorte à la déplacer sur une distance D, le deuxième vérin 39 étant désengagé.

Lors de ce déplacement, la butée 41 a a entraîné la chaîne 32, donc les deux arbres 29, 30 par l'intermédiaire des roues dentées 31, et donc les systèmes à poches 3 associés. Les systèmes à poches 3 se trouvent ainsi dans une position (figure 7b) où la poche primaire 34 se trouve au-dessus de la poche secondaire 36 de sorte que l'écoulement du fluide et donc sa filtration par gravité puissent se produire.

Dans une troisième étape, le premier vérin 38 est désengagé et le deuxième vérin 39 est engagé sur la deuxième butée 41 b. Le premier vérin 38 est déplacé vers sa position initiale alors que le deuxième vérin 39 est déplacé de sorte à compléter le retournement total des systèmes à poches 3 dans le dispositif de manipulation 2 (figure 7c).

On décrit ci-dessous un procédé d'utilisation d'une installation suivant l'invention.

Dans l'exemple d'utilisation décrit, le moteur 13 qui entraîne le câble 12 de la voie principale 8 tourne en continu de sorte que le déplacement des dispositifs de manipulation 2 qui sont engagés dessus par l'intermédiaire des mobiles 16 est réalisé à vitesse continue.

On peut toutefois prévoir que ce déplacement soit effectué à une vitesse et/ou de façon discontinue en régulant la vitesse du moteur 13.

L'utilisation est décrite pour un seul dispositif de manipulation 2 traité par l'installation, il est entendu que ces étapes sont réalisées en continu et de façon parallèle pour un grand nombre de dispositifs de manipulation 2 de sorte que l'installation permet le traitement de 500 à 5000 systèmes à poches 3 simultanément.

Dans une première étape, les systèmes à poches 3 sont chargés dans un dispositif de manipulation 2.

A cet effet, une zone 43 est prévue dans l'installation à laquelle le personnel ayant à effectuer le chargement a un accès facile et dans laquelle le dispositif de manipulation 2 à charger est immobile.

Une telle zone de chargement 43 peut être réalisée par un tronçon de voie non motorisé sur laquelle le dispositif de manipulation 2 se déplaçant sur la voie principale 8 est dévié. Le désengagement du mobile 16 assurant le déplacement du dispositif de manipulation 2 sur la voie principale 8 est réalisé au moyen d'un système d'aiguillage 11.

L'opérateur peut alors charger le dispositif de manipulation 2 immobile avec les systèmes à poches 3 contenant le fluide à filtrer, lesdits systèmes 3 étant disposés dans une position dans laquelle la filtration n'a pas lieu.

Lors de ce chargement, une étape d'identification des systèmes à poches 3 et/ou du dispositif de manipulation 2 est réalisée.

A cet effet, les systèmes à poches 3 et/ou le dispositif de manipulation 2 peuvent être équipés d'un dispositif électronique 44 apte à enregistrer et/ou délivrer des informations de sorte à assurer le suivi du système à poches 3 et/ou de son contenu.

L'opérateur, à l'aide d'un dispositif de lecture/écriture, lit les informations contenues dans le(s) dispositif(s) électronique(s) 44, ledit dispositif de lecture/écriture étant relié à une unité de commande 45 de l'installation, par exemple formée d'un contrôleur électronique.

En fonction des informations lues, l'unité de commande 45 calcule le temps et/ou le lieu de stockage du dispositif de manipulation 2 dans l'installation.

En particulier les informations lues concernent notamment le contenu de la poche primaire 34 et le moment où le remplissage de la poche primaire 34 a été réalisé, à savoir l'heure de prélèvement du fluide. En effet, il est bien connu que la filtration d'un composé sanguin doit être réalisée dans une fourchette de temps prédéterminée après le recueil de celui-ci.

Une fois le chargement et l'identification réalisés, l'opérateur entraîne, par exemple manuellement, le dispositif de manipulation 2 vers un deuxième système d'aiguillage 11 qui permet de remettre ledit dispositif 2 sur la voie principale 8 en engageant un mobile 16 sur le téton 21 afin d'assurer son déplacement le long de ladite voie 8.

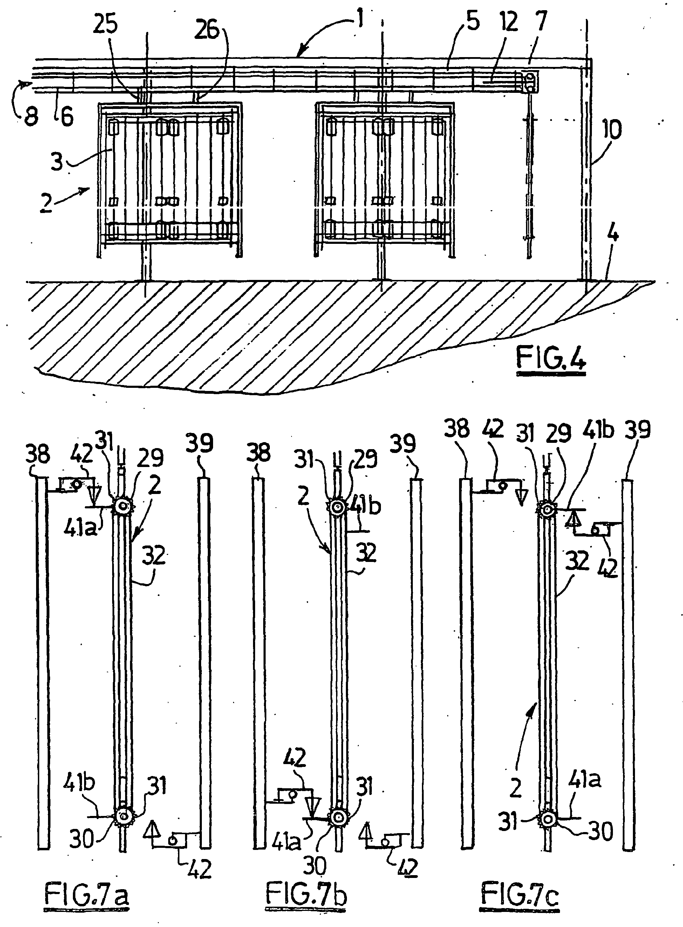

Le dispositif de manipulation 2 est alors déplacé le long de la voie principale 8 pour être amené vers la zone de stockage 9 qui a été déterminée par l'unité de commande 45 (voir figure 4).

Lorsque le dispositif de manipulation 2 arrive sur le système d'aiguillage 11 de la zone de stockage 9 déterminée, l'unité de commande 45 actionne ledit système 11 de sorte que le dispositif de manipulation 2 soit dévié sur la voie secondaire 9 et que le mobile 16 assurant son déplacement soit désengagé.

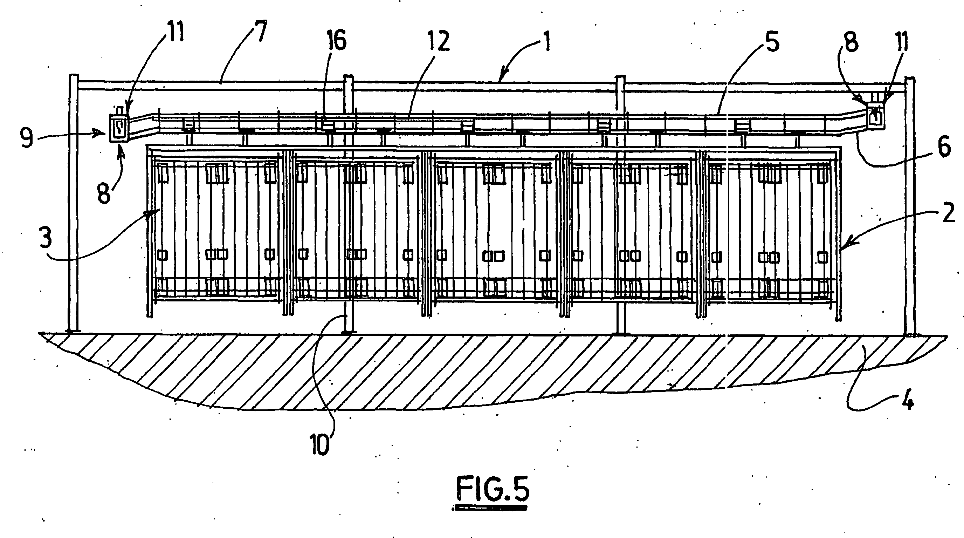

Le dispositif de manipulation 2 se trouve ainsi sur une voie secondaire 9, avec d'autres dispositifs de manipulation 2, dans l'attente d'être actionné, (voir figure 5).

Il est entendu que l'unité de commande 45 règle le flux de dispositifs de manipulation 2 dans les voies secondaires 9 de sorte qu'ils y soient disposés par ordres chronologiques d'heure d'actionnement.

Le déplacement du dispositif d'actionnement 2 dans la voie secondaire 9 de stockage est également réalisé au moyen de mobile 16 qui viennent s'engager et se désengager sur son téton 21.

Dans un exemple particulier, l'actionnement du moteur 13 entraînant le câble 12 des voies secondaires 9 est réalisé de façon discontinue sur commande de l'unité de commande 45 et en fonction des besoins de stockage / déstockage.

Le déstockage du dispositif de manipulation 2 est commandé par l'unité de commande 45, notamment en fonction du temps écoulé depuis le prélèvement.

En variante, des moyens de détermination de la température du fluide (non représentés) sont prévus dans la zone de stockage 9 de sorte à déclencher le déstockage que lorsque la température du fluide est comprise dans la gamme de fonctionnement optimale de l'unité de filtration 35.

Les moyens de détermination sont par exemple de type infrarouge et la gamme de fonctionnement optimale est typiquement comprise entre 18°C et 22°C pour la filtration du sang total.

Lors de l'étape de déstockage, le dispositif de manipulation 2 est déplacé jusqu'à un système d'aiguillage 11 qui permet de remettre ledit dispositif 2 sur la voie principale 8 en venant engager un mobile 16 dessus de sorte à assurer son déplacement jusqu'à une zone d'agitation 46.

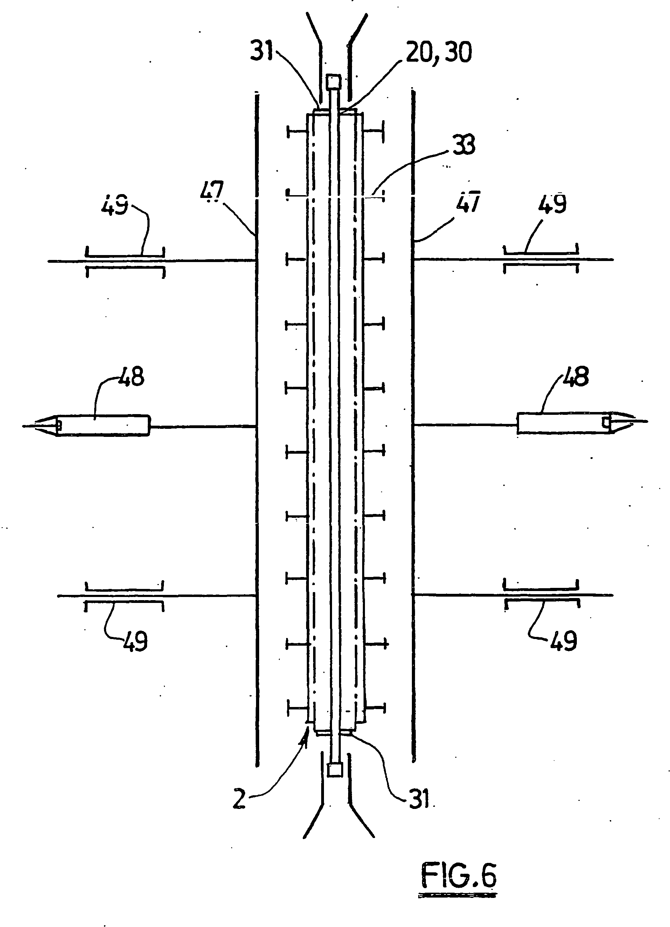

Dans une première variante l'agitation des systèmes à poches est réalisée au moyen d'un système de plaques 47 actionnées par des vérins 48 de sorte à venir presser les poches 34, 36 de façon alternative (voir figure 6).

Dans cette variante, le dispositif de manipulation 2 est immobilisé, par exemple sur un tronçon de voie parallèle à: la voie principale 8 et relié à elle par des systèmes d'aiguillage 11. L'immobilisation est effectuée en regard des plaques 47 puis les vérins 48 sont actionnés afin que les plaques 47 soit déplacées en translation de façon oscillatoire, des moyens de guidage 49 étant prévus, pour assister ces déplacements. Les plaques 47 viennent ainsi presser tes systèmes à poches 3, et notamment les poches primaires 34, afin de remettre en suspension les différents constituants du fluide.

Le dispositif de manipulation 2 est ensuite déplacé vers une zone d'actionnement 40 par l'intermédiaire de la voie principale 8.

Dans une deuxième variante, la zone d'agitation 46 et la zone d'actionnement 40 sont identiques, l'agitation étant réalisée par actionnement partiel du dispositif de manipulation 2.

Dans la zone d'actionnement 40, le dispositif 2 est immobilisé, par exemple sur un tronçon de voie parallèle à la voie principale 8 et relié à elle par des systèmes d'aiguillage 11. L'immobilisation est effectuée en regard des premier et deuxième vérins 38, 39 qui assurent l'actionnement comme décrit ci-dessus.

Dans la deuxième variante, et de façon préalable à l'actionnement complet, un actionnement partiel et alternatif est réalisé de sorte à remettre en suspension les constituants du fluide.

A cet effet, les vérins 38, 39 sont alors de type double effet de sorte à assurer plusieurs retournements partiels des systèmes à poches 3, par exemple entre la position initiale et la moitié de la distance D, et vice versa.

Ces actionnements partiels doivent être réalisés sur une distance telle que le fluide ne rentre pas dans l'unité de filtration 35 pour ne pas initier la filtration.

Une fois l'agitation puis l'actionnement réalisés, le dispositif de manipulation 2 est déplacé le long de la voie principale 8 vers une voie secondaire 9 pour être stocké pendant le temps nécessaire à la filtration de sensiblement tout le fluide.

Dans le cas où le temps de filtration est court et/ou le temps de déplacement depuis la zone d'actionnement 40 vers la zone de déchargement 50 est long, la deuxième zone de stockage peut ne pas être prévue de sorte que la filtration a lieu le long de la voie principale 8.

Lorsque la deuxième zone de stockage est prévue, celle-ci peut être équipée de moyens de détermination de l'achèvement de la filtration (non représentés) qui, étant reliés à l'unité de commande 45, commande la sortie du dispositif 2 de la deuxième zone de stockage. Le dispositif 2 est ensuite déplacé, le long de la voie principale 8, vers la zone de déchargement 50.

De plus, ces moyens de détermination peuvent servir pour détecter d'éventuel problème lors de la filtration, par exemple une filtration trop rapide ou trop lente.

Ces moyens de détermination sont par exemple de type optique pour détecter la présence ou l'absence de liquide dans la tubulure 37, ou pour déterminer l'épaisseur des poches 34, 36.

Une fois la filtration réalisée, le dispositif de manipulation 2 est déplacé jusqu'à la zone de déchargement 50, qui dans le mode de réalisation représenté sur les figures, est la même que la zone de chargement 43.

L'opérateur enlève les systèmes à poche 3 du dispositif de manipulation 2 de sorte à pouvoir utiliser le fluide filtrer de façon conventionnelle.

Lors de cette étape de déchargement, l'opérateur peut également écrire des informations dans le dispositif électronique 44 des systèmes à poches 3 et/ou du dispositif de manipulation 2.

Ces différentes étapes de lecture / écriture d'informations ont pour but d'assurer une bonne tracabilité des fluides traités, notamment toutes les informations peuvent être stockées dans une unité centrale reliée à l'unité de commande 45 de l'installation de sorte à garder la trace de toutes les opérations effectuées.

De plus, la succession des étapes du procédé d'utilisation ainsi que la gestion des flux de dispositifs de manipulation 2 sont réalisées de façon automatique, par l'intermédiaire de l'unité de commande 45, en fonction de ces informations.

## Revendications

1. Procédé de manipulation automatique d'au moins un système à poches (3) dans une installation, ledit système à poches (3) comprenant au moins une poche primaire (34) contenant un fluide, notamment biologique, par exemple du sang ou un composant du sang, au moins une unité de filtration (35) et au moins une poche secondaire (36) destinée à recevoir le filtrat, ledit procédé comprenant les étapes de
- chargement d'au moins un système à poches (3) dans un dispositif de manipulation (2), ledit système à poches (3) étant dans une position telle que la filtration du fluide n'a pas lieu ;
- actionnement automatique du dispositif de manipulation (2) de sorte que le système à poches (3) soit manipulé vers une position dans laquelle le fluide s'écoule par gravité depuis la poche primaire (34) vers la poche secondaire (36) par l'intermédiaire de l'unité de filtration (35), de sorte à permettre la réalisation de la filtration du fluide à un instant prédéterminé ;
- déchargement du système à poches (3) présent dans le dispositif de manipulation (2) lorsque tout le filtrat a été reçu dans la poche secondaire (36).

2. Procédé selon la revendication 1, **caractérisé en ce qu'**il comprend, préalablement à l'actionnement du dispositif de manipulation (2), une étape de stockage du système à poches (3) associé audit dispositif (2) pendant un temps prédéterminé.

3. Procédé selon la revendication 2, **caractérisé en ce que** l'étape de stockage est réalisée par transfert du système à poches (3) associé audit dispositif de manipulation (2) vers une première zone de stockage (9).

4. Procédé selon l'une quelconque des revendications 1 à 3, **caractérisé en ce que** l'étape d'actionnement du dispositif de manipulation (2) est précédée par une étape d'agitation du contenu du système à poches (3), notamment de celui de la poche primaire (34).

5. Procédé selon la revendication 4, **caractérisé en ce que** l'étape d'agitation est réalisée par actionnement partiel du dispositif de manipulation (2).

6. Procédé selon l'une quelconque des revendications 1 à 5, **caractérisé en ce qu'**il comprend, postérieurement à l'actionnement du dispositif de manipulation (2), une étape de stockage du système à poches (3) associé audit dispositif (2) pendant le temps nécessaire à la filtration de sensiblement la totalité du fluide.

7. Procédé selon la revendication 6, **caractérisé en ce que** l'étape de stockage est réalisée par transfert du système à poches (3) associé audit dispositif de manipulation (2) vers une deuxième zone de stockage (9).

8. Procédé selon la revendication 6 ou 7, **caractérisé en ce que** la fin de l'étape de stockage est commandée par des moyens de détermination de l'achèvement de la filtration.

9. Procédé selon l'une quelconque des revendications 1 à 8, **caractérisé en ce qu'**il comprend en outre une ou des étapes d'identification du système à poches (3) et/ou du dispositif de manipulation (2) présent dans l'installation.

10. Procédé selon la revendication 9, **caractérisé en ce que** l'étape d'identification comprend la lecture et/ou l'enregistrement d'informations concernant le dispositif de manipulation (2), le système à poches (3), le contenu de la poche primaire (34), le personnel ayant utilisé le système à poches (3).

11. Procédé selon la revendication 10, **caractérisé en ce que** les informations sont contenues dans un dispositif électronique (44) associé au dispositif de manipulation (2)et/ou au système à poches (3).

12. Procédé selon la revendication 10 ou 11, **caractérisé en ce que** la succession des différentes étapes est commandée en fonction des informations lues lors de l'étape d'identification.

13. Procédé selon l'une quelconque des revendications 10 à 12, **caractérisé en ce qu'**une étape d'identification est réalisée lors du chargement du système à poches (3) dans l'installation de sorte notamment à lire des informations concernant le contenu de la poche primaire (34).

14. Procédé selon la revendication 13, **caractérisé en ce que** l'heure d'actionnement du dispositif de manipulation (2) est commandé en fonction des informations lues lors de l'étape d'identification, notamment en fonction de l'heure de prélèvement.

15. Procédé selon la revendication 14, **caractérisé en ce que** l'actionnement du dispositif de manipulation (2) est également commandé en fonction de la température du fluide à filtrer.

16. Procédé selon l'une quelconque des revendications 9 à 15, **caractérisé en ce qu'**une étape d'identification est réalisée lors du déchargement du système à poches (3) de sorte notamment à enregistrer des informations concernant la réalisation de la filtration.

17. Procédé selon l'une quelconque des revendications 1 à 16, **caractérisé en ce qu'**une pluralité de systèmes à poches (3) est manipulée dans un même dispositif de manipulation (2), par exemple entre 10 et 30 systèmes à poches (3).

18. Procédé selon l'une quelconque des revendications 1 à 17, **caractérisé en ce qu'**une pluralité de système à poches (3) est manipulée simultanément dans une même installation, par exemple entre 500 et 5000 systèmes à poches (3).

19. Installation pour la mise en oeuvre du procédé selon l'une quelconque des revendications 1 à 18, **caractérisée en ce qu'**elle comprend :
- un dispositif de manipulation (2) apte à recevoir au moins un système à poches (3) ;
- des moyens d'actionnement (38,39) aptes à actionner ledit dispositif de manipulation (2) depuis une position dans laquelle la filtration du fluide présent dans le système à poches (3) associé n'a pas lieu vers une position dans laquelle la filtration du fluide a lieu ;
- une zone de chargement (43) des systèmes à poches (3) dans le dispositif de manipulation (2) ;
- une zone de déchargement (50) des systèmes à poches associés au dispositif de manipulation (2) ;
- une zone d'actionnement (40) du dispositif de manipulation (2) dans laquelle les moyens d'actionnement (38,39) sont prévus ;
ladite installation comprenant au moins une voie de circulation principale motorisée (8) sur laquelle le dispositif de manipulation (2) est déplacé depuis la zone de chargement (43) vers la zone de déchargement (50) via la zone d'actionnement (40) et au moins une unité de commande (45) des déplacements du dispositif de manipulation (2) dans l'installation.

20. Installation selon la revendication 19, **caractérisée en ce que** les zones de chargement (43) et de déchargement (50) sont les mêmes.

21. Installation selon la revendication 19 ou 20, **caractérisée en ce qu'**elle comprend en outre, une première et une deuxième zone de stockage dans laquelle le système à poches (3) associé au dispositif de manipulation (2) est stocké respectivement avant et pendant la filtration.

22. Installation selon la revendication 21, **caractérisée en ce que** les zones de stockage sont formées par au moins un tronçon de voie de circulation secondaire motorisée (9).

23. Installation selon l'une quelconque des revendications 19 à 22, **caractérisée en ce qu'**elle comprend en outre une zone d'agitation (46) du contenu du système à poches (3), ladite zone d'agitation (46) étant pourvue de moyens d'agitation.

24. Installation selon la revendication 23, **caractérisée en ce que** la zone d'agitation (46) est identique à celle d'actionnement (40) du dispositif de manipulation (2), les moyens d'actionnement (38,39) formant également moyens d'agitation.

25. Installation selon l'une quelconque des revendications 19 à 24, **caractérisée en ce qu'**elle comprend des moyens de détermination de l'achèvement de la filtration, lesdits moyens de détermination étant reliés à l'unité de commande (45).

26. Installation selon l'une quelconque des revendications 19 à 25, **caractérisée en ce qu'**elle comprend des moyens d'identification du système à poches (3) et/ou du dispositif de manipulation (2) apte à lire et/ou à écrire des informations dans un dispositif électronique (44) associé audit système à poches (3) et/ou audit dispositif de manipulation (2), lesdits moyens d'identification étant reliés à l'unité de commande (45).

27. Installation selon l'une quelconque des revendications 19 à 26, **caractérisée en ce qu'**elle comprend des moyens de détermination de température du fluide à filtrer, lesdits moyens de détermination étant reliés à l'unité de commande (45).

## Claims

1. Method for automatically manipulating at least one pouch system (3) in an installation, said pouch system (3) comprising at least one primary pouch (34) containing a fluid, in particular a biological fluid, for example blood or a blood component, at least one filtration unit (35) and at least one secondary pouch (36) intended to receive the filtrate, said method comprising the steps of
- loading at least one pouch system (3) onto a manipulation device (2), said pouch system (3) being in such a position that the filtration of the fluid does not take place;
- automatically activating the manipulation device (2) so as to place the pouch system (3) in a position in which the fluid flows, by the action of gravity, from the primary pouch (34) towards a secondary pouch (36) through a filtration unit (35), so as to allow filtration of the fluid at a predetermined moment;
- unloading the pouch system (3) from the manipulation device (2) once all the filtrate has been received in the secondary pouch (36).

2. Method according to claim 1, **characterised in that** it comprises, prior to activating the manipulation device (2), a step of storing the pouch system (3) associated with said device (2) for a predetermined time.

3. Method according to claim 2, **characterised in that** the storage step is performed by transferring the pouch system (3) associated with said manipulation device (2) to a first storage area (9).

4. Method according to any one of the claims from 1 to 3, **characterised in that** the step of activating the manipulation device (2) is preceded by a step of agitating the contents of the pouch system (3), in particular those of the primary pouch (34).

5. Method according to claim 4, **characterised in that** the agitation step is performed by partially activating the manipulation device (2).

6. Method according to any one of the claims from 1 to 5, **characterised in that** it comprises, after activating the manipulation device (2), a step of storing the pouch system (3) associated with said device (2) during the time required for filtrating substantially all the fluid.

7. Method according to claim 6, **characterised in that** the storage step is performed by transferring the pouch system (3) associated with said manipulation device (2) to a second storage area (9).

8. Method according to claim 6 or 7, **characterised in that** the end of the storage step is controlled by means for determining when the filtration process is complete.

9. Method according to any one of the claims from 1 to 8, **characterised in that** it also comprises one or more steps of identifying the pouch system (3) and/or the manipulation device (2) of the installation.

10. Method according to claim 9, **characterised in that** the identification step includes reading and/or recording data concerning the manipulation device (2), the pouch system (3), the contents of the primary pouch (34), the persons who have used the pouch system (3).

11. Method according to claim 10, **characterised in that** the data are contained in an electronic device (44) associated with the manipulation device (2) and/or the pouch system (3).

12. Method according to claim 10 or 11, **characterised in that** the succession of different steps is controlled according to the data read during the identification step.

13. Method according to any one of the claims from 10 to 12, **characterised in that** an identification step is performed when loading the pouch system (3) onto the installation, in particular in order to read data concerning the contents of the primary pouch (34).

14. Method according to claim 13, **characterised in that** the time at which the manipulation device (2) is activated is controlled according to the data read during the identification step, in particular according to the time of sampling.

15. Method according to claim 14, **characterised in that** the activation of the manipulation device (2) is also controlled according to the temperature of the fluid to be filtrated.

16. Method according to any one of the claims from 9 to 15, **characterised in that** an identification step is performed when unloading the pouch system (3) in order to record, in particular, data concerning the performance of the filtration.

17. Method according to any one of the claims from 1 to 16, **characterised in that** a plurality of pouch systems (3) are manipulated in the same manipulation device (2), for example between 10 and 30 pouch systems (3).

18. Method according to any of the claims from 1 to 17, **characterised in that** a plurality of pouch systems (3) are simultaneously manipulated in the same installation, for example between 500 and 5000 pouch systems (3).

19. Installation for implementing the method according to any one of the claims from 1 to 18, **characterised in that** it comprises:
- a manipulation device (2) capable of receiving at least one pouch system (3);
- activation means (38, 39) capable of activating said manipulation device (2) from a position in which no filtration of the fluid contained in the associated pouch system (3) takes place to a position in which the fluid filtration takes place;
- an area (43) for loading pouch systems (3) onto the manipulation device (2);
- an area (50) for unloading pouch systems associated with the manipulation device (2);
- an area (40) for activating the manipulation device (2) in which the activation means (38, 39) are provided;
said installation comprising at least one main motorised circulation path (8) on which the manipulation device (2) is moved from the loading area (43) to the unloading area (50) via the activation area (40), and at least one unit (45) for controlling the movement of the manipulation device (2) in the installation.

20. Installation according to claim 19, **characterised in that** the loading (43) and unloading (50) areas are the same.

21. Installation according to claim 19 or 20, **characterised in that** it also comprises a first and second storage area in which the pouch system (3) associated with the manipulation device (2) is stored before and after filtration, respectively.

22. Installation according to claim 21, **characterised in that** the storage areas are formed by at least one section of a secondary motorised circulation path (9).

23. Installation according to any one of the claims from 19 to 22, **characterised in that** it also comprises an area (46) for agitating the contents of the pouch system (3), said agitation area (46) being equipped with agitation means.

24. Installation according to claim 23, **characterised in that** the agitation area (46) is identical to the activation area (40) of the manipulation device (2), the activation means (38, 39) also constituting agitation means.

25. Installation according to any one of the claims from 19 to 24, **characterised in that** it comprises means for determining when the filtration process is complete, said determination means being connected to the control unit (45).

26. Installation according to any one of the claims from 19 to 25, **characterised in that** it comprises means for identifying the pouch system (3) and/or manipulation device (2), which are capable of reading and/or writing data from/to an electronic device (44) associated with said pouch system (3) and/or said manipulation device (2), said identification means being connected to the control unit (45).

27. Installation according to any one of the claims from 19 to 26 **characterised in that** it comprises means for determining the temperature of the fluid to be filtrated, said determination means being connected to the control unit (45).

## Patentansprüche

1. Verfahren zum automatischen Manipulieren wenigstens eines Beutelsystems (3) in einer Einrichtung, wobei besagtes Beutelsystem (3) wenigstens einen primären Beutel (34) mit einer insbesondere biologischen Flüssigkeit, zum Beispiel Blut oder einen Blutbestandteil, wenigstens eine Filtriereinrichtung (35) und wenigstens einen sekundären Beutel (36) zur Aufnahme des Filtrats enthält, wobei das besagte Verfahren folgende Stufen umfasst:
- Laden wenigstens eines Beutelsystems (3) in eine Manipuliereinrichtung (2), wobei besagtes Beutelsystem (3) sich in einer solchen Position befindet, dass das Filtrieren der Flüssigkeit nicht stattfindet;
- automatische Betätigung der Manipuliereinrichtung (2) auf solche Weise, dass das Beutelsystem (3) in eine Position manipuliert wird, in der die Flüssigkeit durch Schwerkraft von dem primären Beutel (34) in den sekundären Beutel (36) auf solche Weise durch die Filtriereinrichtung (35) abläuft, dass die Realisierung der Filtrierung zu einem vorbestimmten Zeitpunkt stattfinden kann;
- Entladen des in der Manipuliereinrichtung (2) vorhandenen Beutelsystems (3), wenn das gesamte Filtrat im sekundären Beutel (36) aufgefangen worden ist.

2. Verfahren nach Anspruch 1, **dadurch gekennzeichnet, dass** es vor der Betätigung der Manipuliereinrichtung (2) eine Lagerungsstufe des der genannten Einrichtung (2) während einer vorbestimmten Zeit zugeordneten Beutelsystems (3) umfasst.

3. Verfahren nach Anspruch 2, **dadurch gekennzeichnet, dass** die Lagerungsstufe per Transfer des der Manipuliereinrichtung (2) zu einer ersten Lagerungszone (9) zugeordneten Beutelsystems (3) realisiert wird.

4. Verfahren nach Anspruch 1 bis 3, **dadurch gekennzeichnet, dass** der Betätigungsstufe der Manipuliereinrichtung (2) eine Schüttelstufe des Inhalts des Beutelsystems (3) vorausgeht, insbesondere des Systems mit dem primären Beutel (34).

5. Verfahren nach Anspruch 4, **dadurch gekennzeichnet, dass** die Schüttelstufe durch teilweises Betätigen der Manipuliereinrichtung (2) realisiert wird.

6. Verfahren nach Anspruch 1 bis 5, **dadurch gekennzeichnet, dass** es nach der Betätigung der Manipuliereinrichtung (2) eine Lagerungsstufe des der besagten Einrichtung (2) während der zur Filtrierung deutlich der gesamten Flüssigkeit notwendigen Zeit zugeordneten Beutelsystems (3) umfasst.

7. Verfahren nach Anspruch 6, **dadurch gekennzeichnet, dass** die Lagerungsstufe per Transfer des der besagten Manipuliereinrichtung (2) zu einer zweiten Lagerungszone (9) zugeordneten Beutelsystems (3) realisiert wird.

8. Verfahren nach Anspruch 6 oder 7, **dadurch gekennzeichnet, dass** das Ende der Lagerungsstufe durch Bestimmungsmittel des Abschlusses der Filtrierung gesteuert wird.

9. Verfahren nach Anspruch 1 bis 8, **dadurch gekennzeichnet, dass** es weiterhin eine oder mehrere Identifizierungsstufen des Beutelsystems (3) und / oder der in der Anlage vorhandenen Manipuliereinrichtung (2) umfasst.

10. Verfahren nach Anspruch 9, **dadurch gekennzeichnet, dass** die Identifizierungsstufe das Lesen und / oder das Speichern von Informationen bezüglich der Manipuliereinrichtung (2), des Beutelsystems (3) und des Inhaltes des primären Beutels (34) umfasst, wobei das Personal das Beutelsystem (3) benutzt hat.

11. Verfahren nach Anspruch 10, **dadurch gekennzeichnet, dass** die Informationen in einer der Manipuliereinrichtung (2) und / oder dem Beutelsystem (3) zugeordneten elektronischen Vorrichtung (44) enthalten sind.

12. Verfahren nach Anspruch 10 oder 11, **dadurch gekennzeichnet, dass** die Folge der unterschiedlichen Stufen in Abhängigkeit von den bei der Identifizierungsstufe abgelesenen Informationen gesteuert wird.

13. Verfahren nach Anspruch 10 bis 12, **dadurch gekennzeichnet, dass** eine Identifizierungsstufe beim Laden des Beutelsystems (3) in eine Anlage derart realisiert wird, dass insbesondere Informationen bezüglich des Inhalts des primären Beutels (34) gelesen werden.

14. Verfahren gemäß Anspruch 13, **dadurch gekennzeichnet, dass** die Betätigungszeit der Manipuliereinrichtung (2) in Abhängigkeit von den bei der Identifizierungsstufe abgelesenen Informationen, insbesondere in Abhängigkeit von der Entnahmezeit gesteuert wird.

15. Verfahren nach Anspruch 14, **dadurch gekennzeichnet, dass** die Betätigung der Manipuliereinrichtung (2) ebenfalls in Abhängigkeit von der Temperatur der zu filternden Flüssigkeit gesteuert wird.

16. Verfahren nach Anspruch 9 bis 15, **dadurch gekennzeichnet, dass** eine Identifizierungsstufe beim Entladen des Beutelsystems (2) derart realisiert wird, dass insbesondere Informationen bezüglich der Realisierung der Filtrierung gespeichert werden.

17. Verfahren gemäß Anspruch 1 bis 16, **dadurch gekennzeichnet, dass** eine Vielzahl von Beutelsystemen (3), z. B. zwischen 10 und 30 Beutelsystemen (3) in einer und derselben Manipuliereinrichtung (2) manipuliert wird.

18. Verfahren gemäß Anspruch 1 bis 7, **dadurch gekennzeichnet, dass** eine Vielzahl von Beutelsystemen (3), z. B. zwischen 500 und 5.000 Beutelsystemen (3) gleichzeitig in einer und derselben Anlage manipuliert wird.

19. Anlage zur Umsetzung des Verfahrens gemäß Anspruch 1 bis 18, **dadurch gekennzeichnet, dass** sie Folgendes umfasst:
- eine zur Aufnahme wenigstens eines Beutelsystems (3) geeignete Manipuliereinrichtung (2);
- zur Betätigung der besagten Manipuliereinrichtung (2) ausgehend von einer Position geeignete Betätigungsmittel (38, 39), in der die Filtrierung der im zugeordneten Beutelsystem (3) vorhandenen Flüssigkeit zu einer Position, in der die besagte Filtrierung der Flüssigkeit stattfindet, nicht stattfindet;
- einen Beladebereich (43) der Beutelsysteme (3) in der Manipuliereinrichtung (2);
- einen Entladebereich (50) der der Manipuliereinrichtung (2) zugeordneten Beutelsysteme;
- einen Betätigungsbereich (40) der Manipuliereinrichtung (2), in der die Betätigungsmittel (38, 39) vorgesehen sind;
wobei besagte Einrichtung wenigstens einen motorisierten Hauptverkehrsweg (8) umfasst, auf dem die Manipuliereinrichtung (2) von dem Beladebereich (43) zum Entladebereich (50) über den Betätigungsbereich (40) verschiebbar ist, und wenigstens eine Steuereinheit (45) der Verschiebungen der Manipuliereinrichtung (2) in der Anlage.

20. Anlage nach Anspruch 19, **dadurch gekennzeichnet, dass** die Belade- (43) und Entladebereiche (50) identisch sind.

21. Anlage nach Anspruch 19 oder 20, **dadurch gekennzeichnet, dass** sie weiterhin eine erste und zweite Lagerungszone umfasst, in der das der Manipuliereinrichtung (2) zugeordnete Beutelsystem (3) jeweils vor und während der Filtrierung gelagert wird.

22. Anlage nach Anspruch 21, **dadurch gekennzeichnet, dass** die Lagerungszonen durch wenigstens einen Abschnitt eines motorisierten Nebenverkehrsweges gebildet werden.

23. Anlage nach Anspruch 19 bis 22, **dadurch gekennzeichnet, dass** sie weiterhin eine Schüttelzone (46) des Inhaltes des Beutelsystems (3) umfasst, wobei die besagte Schüttelzone (46) mit Schüttelmitteln versehen ist.

24. Anlage nach Anspruch 23, **dadurch gekennzeichnet, dass** die Schüttelzone (46) mit der des Betätigungsbereichs (40) der Manipuliereinrichtung (2) identisch ist, wobei die Betätigungsmittel (38, 39) ebenfalls Betätigungsmittel bilden.

25. Anlage gemäß Anspruch 19 bis 24, **dadurch gekennzeichnet, dass** sie Bestimmungsmittel des Abschlusses der Filtrierung umfasst, wobei die besagten Bestimmungsmittel mit der Steuereinheit (45) verbunden sind.

26. Anlage nach Anspruch 19 bis 25, **dadurch gekennzeichnet, dass** sie Identifizierungsmittel des Beutelsystems (3) und /oder der Manipuliereinrichtung (2) umfasst, die geeignet sind, Informationen in eine dem Beutelsystem (3) und oder der besagten Manipuliereinrichtung (2) zugeordnete elektronische Vorrichtung (44) zu schreiben, wobei die besagten Identifizierungsmittel mit der Steuereinheit (45) verbunden sind.

27. Anlage nach Anspruch 19 bis 26, **dadurch gekennzeichnet, dass** sie Temperaturbestimmungsmittel der zu filtrierenden Flüssigkeit umfasst, wobei die besagten Bestimmungsmittel mit der Steuereinheit (45) verbunden sind.
